(19) Europäisches Patentamt
European Patent Office
Office européen des brevets

(11) Numéro de publication : **0 468 856 B1**

# (12) FASCICULE DE BREVET EUROPEEN

(45) Date de publication du fascicule du brevet : **17.02.93 Bulletin 93/07**

(51) Int. Cl.[5] : **C07C 323/60,** C11D 1/00, A61K 7/043, A61K 7/06, A61K 7/48, B01F 17/18

(21) Numéro de dépôt : **91401988.0**

(22) Date de dépôt : **16.07.91**

## (54) Composés solubilisants et/ou dispersants, procédé de préparation et compositions les contenant.

(30) Priorité : **25.07.90 FR 9009532**

(43) Date de publication de la demande : **29.01.92 Bulletin 92/05**

(45) Mention de la délivrance du brevet : **17.02.93 Bulletin 93/07**

(84) Etats contractants désignés : **AT BE CH DE DK FR GB IT LI NL SE**

(56) Documents cités :
**EP-A- 0 071 993**
**DE-A- 2 753 095**
**US-A- 4 242 516**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**

(74) Mandataire : **Casalonga, Alain et al**
**BUREAU D.A. CASALONGA - JOSSE**
**Morassistrasse 8**
**W-8000 München 5 (DE)**

EP 0 468 856 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés tensio-actifs quaternaires, leur procédé de préparation et des compositions cosmétiques et dermopharmaceutiques en comportant.

Dans les compositions cosmétiques et dermopharmaceutiques aqueuses ou hydro-alcooliques, il est souvent nécessaire d'utiliser des agents solubilisants pour des produits insolubles dans l'eau et il est connu d'utiliser dans cette optique des sels d'ammonium quaternaires. Les sels d'ammonium quaternaires connus peuvent présenter certains inconvénients, notamment au niveau de leur tolérance cutanée.

La demanderesse a découvert de nouveaux composés tensio-actifs quaternaires ayant de bonnes propriétés solubilisantes ou dispersantes en milieux aqueux et hydro-alcooliques et présentant d'une façon tout à fait surprenante une très nette amélioration des propriétés au niveau de la tolérance cutanée comparativement aux ammonium quaternaires connus.

Un objet de l'invention est donc constitué par des composés tensio-actifs quaternaires à chaîne grasse comportant à l'autre extrémité de la chaîne grasse, en position ω par rapport au groupement ammonium, un groupe carboxylique.

Un autre objet de l'invention est constitué par un procédé de préparation des nouveaux composés tensio-actifs quaternaires.

En outre, l'invention concerne des compositions de traitement des matières kératiniques, destinées à l'application topique et contenant les tensio-actifs quaternaires selon l'invention, ainsi que l'utilisation de ces tensio-actifs quaternaires dans un support approprié pour le traitement cosmétique ou dermopharmaceutique des matières kératiniques.

D'autres objets de l'invention apparaîtront à la lecture de la description et des exemples qui suivent.

Les composés selon l'invention sont les tensio-actifs quaternaires représentés par la formule générale (I) :

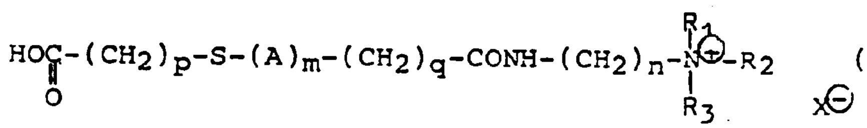 (I)

dans laquelle :

$R_1$ et $R_2$, identiques ou différents, désignent un radical méthyle ou éthyle;

$R_3$ désigne un radical méthyle, éthyle ou hydroxyéthyle;

A désigne un radical

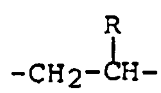

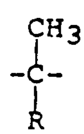

ou

R désigne un hydrogène ou un radical méthyle;

$X^-$ désigne $Cl^-$,$Br^-$,$I^-$,$CH_3OSO_3^-$,$CH_3SO_3^-$,

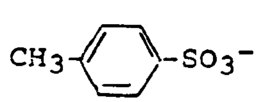

ou $C_2H_5OSO_3^-$;

p et q, identiques ou différents, désignent des nombres entiers tels que $1 \leqq p \leqq 15$, $0 \leqq q \leqq 13$ et $2 \leqq p+q \leqq 20$ ;

n désigne un nombre entier égal à 2 ou 3;

m désigne 0 ou 1, et $m + q \neq 0$

ainsi que les sels de neutralisation par des bases.

Les bases de neutralisation du groupement carboxyle, de préference cosmétiquement ou pharmaceutiquement acceptables, peuvent être notamment choisies parmi la soude, la potasse, l'ammoniaque, la magnésie, la diméthyléthanolamine, les aminométhylpropanols, les aminométhylpropanediols, notamment l'amino-2 méthyl-2 propanol-1 et l'amino-2 méthyl-2 propanediol-1,3, la triéthanolamine, et la N-méthylglucamine.

Les composés préférés répondant à la formule (I) sont ceux pour lesquels $R_1$ et/ou $R_2$ représentent le radical méthyle.

D'autres composés préférés de formule (I) sont ceux pour lesquels n représente 3, q représente 10 et m représente 0.

Les composés particulièrement préférés sont le méthylsulfate de carboxy-10"décylthio-11'undécanoyl amino-3 propyltriméthylammonium et le méthylsulfate de carboxyméthylthio-11'undécanoylamino-3 propyltriméthyl ammonium.

Un autre objet de l'invention est constitué par le procédé de préparation des tensio-actifs quaternaires carboxyliques selon l'invention.

Ils peuvent être préparés à partir de mercapto acides de formule (II) :

$$HOOC - (CH_2)_p - SH \quad (II)$$

par réaction d'addition sur des dérivés insaturés de formule (III) :

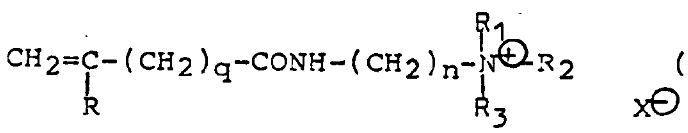

(III)

dans lesquelles $R_1$, $R_2$, $R_3$, R, $X^-$, n, p et q ont les mêmes significations que précédemment, éventuellement en présence de catalyseur dans un solvant approprié.

Par le jeu usuel du choix d'un solvant approprié et des conditions réactionnelles, on peut orienter l'addition sur l'un ou l'autre des deux carbones de la liaison insaturée.

Dans le cas où R représente un radical méthyle, un composé particulièrement préféré de formule (III) est le chlorure de méthyl-2'propenoylamino-3 propyltriméthylammonium, de formule :

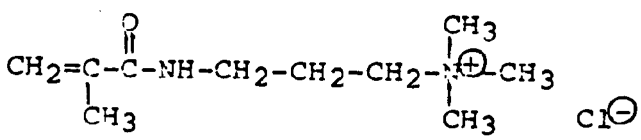

dont l'amine correspondante est commerciale.

La réaction d'addition peut être de type radicalaire, elle a alors lieu en milieu solvant en présence d'initiateur de radicaux libres.

Comme initiateurs de radicaux, on peut citer notamment les hydroperoxydes tels que l'hydroperoxyde de tertiobutyle, les peroxydes tels que le peroxyde de dibenzoyle, les peresters tels que le peroxybenzoate de tertiobutyle ou les dérivés azoïques, et en particulier l'azobis isobutyronitrile. La plupart des solvants peuvent être utilisés notamment l'éthanol, le toluène ou le tétrahydrofuranne.

L'ammonium quaternaire de formule (III) est dissous dans le solvant en présence d'un initiateur de radicaux puis une quantité sensiblement équimolaire d'un mercaptoacide de formule (II) est introduite, la réaction étant de préférence réalisée sous atmosphère inerte.

La réaction d'addition peut se faire aussi par voie ionique en milieu basique. On obtient dans ce cas des isomères issus de l'addition sur l'un ou l'autre carbone éthylénique.

Les composés de formule (I) où m est égal à 0, peuvent être également obtenus par réaction de condensation des mêmes mercaptoacides de formule (II) avec des dérivés halogénés de formule (IV) :

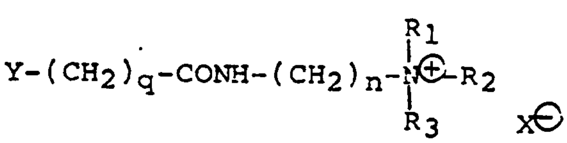

(IV)

dans lesquelles $R_1$, $R_2$, $R_3$, $X^-$, n, p et q ont les mêmes significations que précédemment et Y désigne Cl ou

Br, de préférence Br.

Les mercaptoacides de formule (II) peuvent être préparés à partir de l'acide homologue insaturé en bout de chaîne par réaction avec l'acide thioacétique, comme cela apparaîtra dans les exemples pour le cas particulier où p représente 10.

Quelle que soit la variante utilisée pour synthétiser les tensio-actifs quaternaires selon l'invention, la réaction d'addition ou de condensation peut être suivie d'une neutralisation totale ou partielle par une ou plusieurs bases, choisies notamment dans le groupe évoqué ci-dessus.

Les composés de formule (I) se présentent généralement sous la forme de poudres blanches, solubles dans l'eau à la température ambiante, ou donnant des solutions nacrées qui se clarifient en tiédissant.

Les propriétés de solubilité et de viscosité varient notamment selon les valeurs de p et q et en fonction du pH et de la base éventuellement utilisée pour neutraliser le groupement carboxyle.

Les composés de l'invention possèdent de très bonnes propriétés dispersantes et/ou solubilisantes en milieu aqueux pour des produits non solubles ou difficilement solubles en milieux aqueux ou hydroalcooliques. C'est pourquoi l'invention a pour objet l'utilisation des composés de formule (I) comme solubilisants et/ou dispersants dans des compositions aqueuses ou hydroalcooliques. Ces produits difficilement ou non solubles peuvent être par exemple des colorants pour cheveux, des cires, des polymères, des agents actifs cosmétiques ou dermopharmaceutiques et notamment quand ils sont à caractère basique. Cela apparaîtra dans les exemples ci-après.

Les composés de l'invention présentent par ailleurs de bonnes propriétés cosmétiques. Ils permettent, en effet, par exemple, d'apporter de la douceur, en particulier sur les cheveux, ce qui facilite alors leur démêlage. Ils peuvent être utilisés ainsi pour leurs propriétés adoucissantes.

Compte tenu de leurs propriétés de solubilisation ou dispersion dans l'eau de produits généralement difficiles à formuler, de leurs propriétés cosmétiques et de leurs excellentes propriétés toxicologiques, les tensioactifs quaternaires selon l'invention sont particulièrement intéressants comme additifs dans les compositions pour l'application topique et notamment dans les compositions de traitement des matières kératiniques. Par matières kératiniques selon l'invention, on entend les cheveux, les poils, les ongles et la peau.

L'invention a donc également pour objet l'utilisation des tensio-actifs quaternaires de formule (I) comme additifs dans des compositions de traitement des matières kératiniques.

La forme acide des composés de formule (I) est le plus souvent préférée. Il est toutefois fréquent d'utiliser dans une même composition les composés (I) et leurs sels de neutralisation par des bases, notamment par les bases évoquées ci-dessus qui sont utilisées pour ajuster le pH des compositions en neutralisant au moins partiellement le groupement acide carboxylique.

L'invention concerne donc des compositions aqueuses ou hydroalcooliques en vue de leur application topique, caractérisée en ce qu'elles comprennent au moins un composé de formule (I) selon l'invention, ou leurs sels de neutralisation avec des bases.

Les compositions selon l'invention comportent de 0,1 à 10% en poids de composés de formule (I), et de préférence de 0,3 à 5% en poids.

Les compositions selon l'invention peuvent être utilisées pour la coloration fugace ou semi-permanente des cheveux, pour le maquillage ou le démaquillage, pour le traitement des cheveux en vue d'améliorer le démêlage ou la mise en plis, pour le traitement préventif ou curatif des pellicules, pour la repousse des cheveux ou pour empêcher leur chute, pour le traitement de la peau en vue de combattre l'acné ou de toute autre maladie infectieuse.

Selon leur utilisation en cosmétique ou en dermopharmacie, en plus des produits tensio-actifs quaternaires de formule (I), ou leurs sels, les compositions selon l'invention peuvent en outre contenir des agents actifs dans un excipient approprié.

Ainsi, pour être appropriées pour une application topique, elles peuvent comprendre de l'eau, un mélange d'eau avec un ou plusieurs solvant(s) organique(s) et/ou avec un ou plusieurs corps gras cosmétiquement et dermopharmaceutiquement acceptables.

Les compositions selon l'invention peuvent se présenter notamment sous la forme de solution, de dispersion, de lait, de crème, de mousse, de gel, de pain, de savon ainsi que sous forme de spray.

Ces compositions peuvent éventuellement être pressurisées dans des dispositifs aérosols, en présence d'un agent propulseur, éventuellement en présence de générateurs de mousse ou d'agents émulsionnants.

Comme agents propulseurs, on peut envisager des agents du type fréons, alcanes en $C_3$ à $C_5$, chlorure de méthylène ou diméthyléther.

Les solvants utilisables sont choisis parmi les alcools inférieurs en $C_2$ à $C_5$, tels que l'alcool éthylique ou l'alcool isopropylique, les éthers de glycols, parmi lesquels on peut citer les monoalkyléthers de monoéthylèneglycol, les monoalkyléthers de dialkylèneglycol, les monoalkyléthers de triéthylèneglycol, dans lesquels le groupement alkyle a de préférence 1 à 4 atomes de carbone, tel que par exemple le monoéthyléther d'éthylèneglycol, le monobutyléther d'éthylèneglycol, le monoéthyléther de diéthylèneglycol.

Les compositions selon l'invention peuvent de plus comporter une ou plusieurs huiles minérales, animales, végétales ou synthétiques, en particulier les huiles de silicone, un ou plusieurs polymères de synthèse ioniques ou non-ioniques, un ou plusieurs polymères d'origine naturelle, cellulosiques ou dérivés de chitine ou chitosane.

Le milieu approprié pour une application topique peut être épaissi ou non. Pour l'épaissir, on peut utiliser les agents épaississants ou gélifiants bien connus dans l'état de la technique, tels que par exemple la gomme de guar, les hétérobiopolysaccharides, tels que la gomme de xanthane et les scléroglucanes, les dérivés de cellulose, tels que la méthylcellulose, l'hydroxyéthylcellulose, l'hydroxypropylméthylcellulose, les sels de sodium de la carboxyméthylcellulose et les polymères d'acides acryliques préférentiellement réticulés.

D'autres tensio-actifs ioniques ou non ioniques, des agents moussants ou émulsionnants peuvent également être utilisés.

Comme agents actifs, les compositions selon l'invention peuvent contenir notamment des colorants usuels pour les matières kératiniques, des filtres solaires, des conservateurs, des agents actifs pour la repousse des cheveux ou pour limiter leur chute, comme le minoxidil, des agents actifs pour le traitement des pellicules, des agents actifs pour le traitement de l'acné.

En outre, les compositions selon l'invention peuvent comporter des nacrants ou des agents hydratants et des agents conservateurs ou régulateurs du pH, tels que des acides, des bases, des sels minéraux ou des stérols.

L'invention a en outre pour objet l'utilisation des compositions telles que définies ci-dessus, pour le traitement cosmétique des matières kératiniques et notamment des cheveux, poils, ongles ou de la peau.

Les exemples suivants sont destinés à illustrer l'invention sans pour autant présenter un caractère limitatif.

PREPARATION DU COMPOSE A

Méthylsulfate de carboxy-10"décylthio-11' undécanoyl amino-3 propyltriméthylammonium (A).

I. PREPARATION DE L'ACIDE MERCAPTO-11 UNDECANOIQUE.

a) MODE OPERATOIRE

On réalise la synthèse schématisée ci-après :

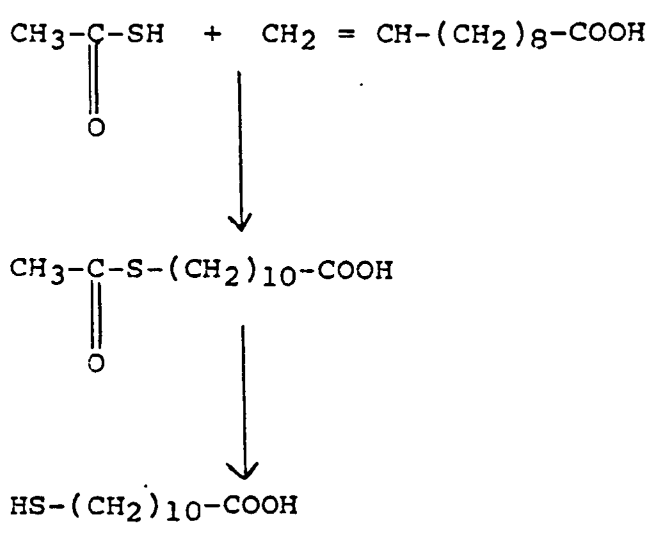

On introduit dans un réacteur :

- 92 g d'acide undécénoïque ( purum " Fluka" à 100%)
- 1, 3 g d'azobis isobutyronitrile ( purum "Fluka" à 97%).

On purge soigneusement à l'azote et on chauffe à 60°C. On introduit régulièrement en 75 minutes, 30 g d'acide thioacétique; la réaction est exothermique et la température atteint 70°C.

Après l'addition, on maintient à 70°C pendant 4 heures supplémentaires, sous agitation et sous azote.

Au milieu réactionnel précédent, ramené à 40°C, on ajoute en 10 minutes une solution de 66,7 g de potasse mise en solution dans 25 ml d'eau et 180 ml d'éthanol à 96°. On porte alors au reflux et on maintient à cette température pendant 3 heures, sous atmosphère d'azote.

Après arrêt du chauffage, on élimine l'éthanol par mise du réacteur sous pression réduite, puis on ajoute 100 ml d'eau.

A la solution précédente, maintenue à 50°C, on ajoute en 30 minutes une solution de 106 g d'acide chlorhydrique concentré à 35%, dans 100 g d'eau.

L'acide formé lors de la neutralisation surnage sous forme huileuse.

Le mélange, toujours sous atmosphère d'azote, est laissé en agitation pendant 1 heure; après décantation, la phase aqueuse (acide) est éliminée, la phase organique est lavée trois fois à 60°C par 150 ml d'eau, récupérée et concentrée.

On obtient 105 g d'un produit huileux qui se solidifie à la température ambiante. Le rendement est de 96%.

La pureté est de 87% (dosage du thiol).

b) ANALYSES DU PRODUIT OBTENU.

Le produit peut être en outre purifié par distillation moléculaire:

Fusion à : 52,5°C

Analyse élémentaire : $C_{11}H_{22}O_2S$ PM = 218,4

| | C | H | O | S |
|---|---|---|---|---|
| Théorique | 60,55 | 10,09 | 14,68 | 14,68 |
| Trouvé | 60,64 | 10,16 | 14,38 | 14,68 |

II. METHYLSULFATE DE CARBOXY-10"DECYLTHIO-11' UNDECANOYL AMINO-3 PROPYLTRIMETHYLAMMONIUM (A).

a) MODE OPERATOIRE

On réalise la synthèse schématisée ci-après :

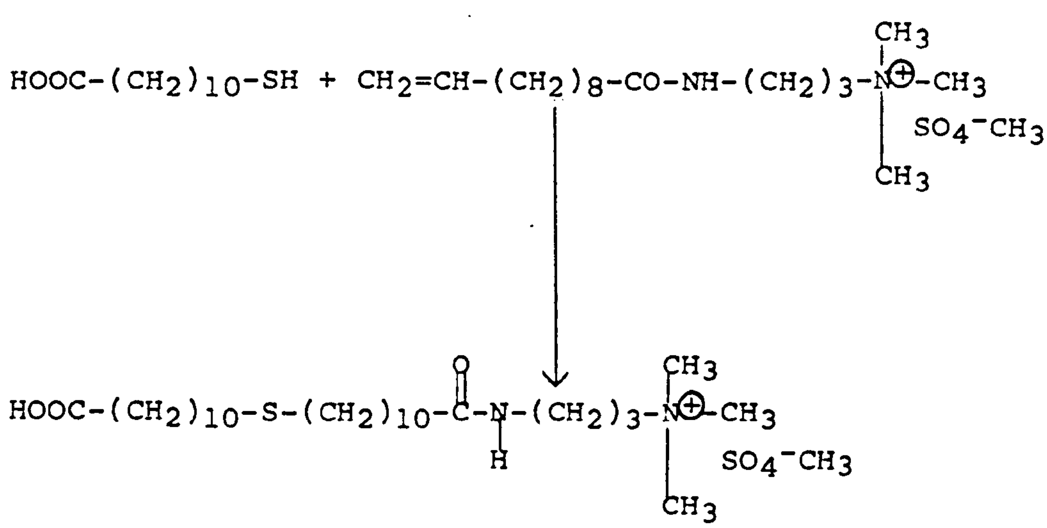

On introduit dans un réacteur soigneusement purgé à l'azote :

- 0,1 mole de méthylsulfate d'undécène-10' oylamino-3 propyltriméthylammonium en solution dans l'eau (47% d'actif), soit 83,8 g de solution.
- 0,64 g d'azobis isobutyronitrile
- 25 g d'éthanol à 96°.

L'acide mercapto-11 undécanoïque est utilisé brut avant distillation. Son taux en thiol est de 86,7%. Le méthylsulfate d'undécène-10'oylamino-3 propyl triméthylammonium que l'on trouve dans le commerce sous la marque "REWOCID UTM 185" commercialisé par la Société REWO en solution dans l'eau à environ 50% est dosé à 47% d'actif.

Le mélange est chauffé à 50°C; à la solution limpide, sous atmosphère d'azote, on ajoute en 5 minutes, 0,1 mole d'acide mercapto-11 undécanoïque pur, fondu, soit 25,1 g d'acide brut à 86,7%.

On chauffe alors à 70°C et on maintient à cette température pendant 2 h 30 minutes sous agitation et sous azote. Le taux de réaction, déterminé par dosage du thiol résiduel, est alors voisin de 95%.

On ajoute 0,16 g d'azobis-isobutyronitrile et on poursuit le chauffage pendant 3 heures, tout en distillant l'éthanol, à la pression ordinaire.

En fin de réaction, on arrête le chauffage et on poursuit l'élimination des solvants par mise du réacteur sous pression réduite.

Le mélange réactionnel résiduel est dissous à chaud dans 300 g d'acétone.

Le produit attendu recristallise par refroidissement de la solution filtrée, on l'essore et on le lave à l'acétone refroidie.

Après séchage, 56 g de poudre blanche (Rendement = 86,8%) sont obtenus.

b) ANALYSES DU PRODUIT OBTENU

- Fusion à : 115°C
- RMN $^{13}C$ : conforme à la formule.
- Analyse élémentaire : $C_{29}H_{60}H_2O_7S_2$ PM = 612

| | C | H | O | N | S |
|---|---|---|---|---|---|
| Théorique | 56,86 | 9,81 | 18,30 | 4,57 | 10,46 |
| Trouvé | 56,72 | 9,84 | 18,26 | 4,51 | 10,36 |

PREPARATION DU COMPOSE B

Méthylsulfate de carboxyméthylthio-11'undécanoylamino-3 propyltriméthylammonium (B).

a) MODE OPERATOIRE

On réalise la synthèse schématisée ci-après :

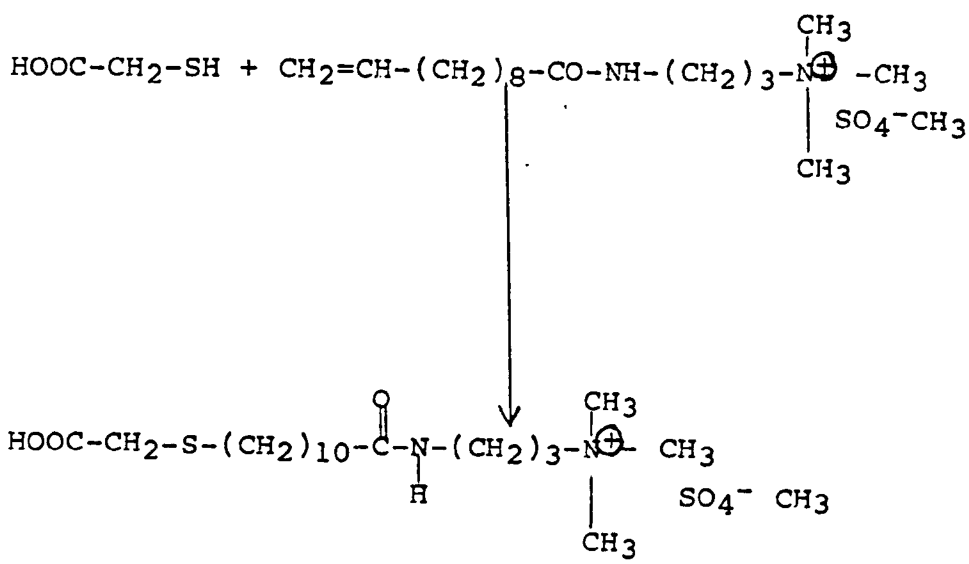

On introduit dans un réacteur soigneusement purgé à l'azote :

- 0,8 mole de méthylsulfate d'undécène-10′ oylamino-3 propyltriméthylammonium en solution dans l'eau (47% d'actif), soit 670,6 g de solution.
- 3,9 g d'azobis isobutyronitrile
- 220 g d'éthanol à 96°.

L'acide mercapto acétique utilisé est dosé à 97% d'actif. Le méthylsulfate d'undécène-10′oylamino-3 propyltriméthylammonium utilisé est comparable à celui utilisé à l'exemple de préparation A.

Le mélange est chauffé à 55°C; à la solution limpide, sous atmosphère d'azote, on ajoute en 30 minutes, 0,8 mole d'acide mercaptoacétique (97% d'actif), soit 75,9 g.

On chauffe alors à 65°C et on maintient à cette température pendant 3 h 30 minutes sous agitation et sous azote. Le taux de réaction, déterminé par dosage du thiol résiduel, est alors voisin de 95%.

On monte la température à 80°C et on distille en 2 heures les solvants (200 g distillés).

En fin de réaction, on arrête le chauffage et on poursuit l'élimination des solvants par mise du réacteur sous pression réduite, de la même façon qu'à l'exemple A.

Le mélange réactionnel résiduel est dissous à chaud dans 2,5 l d'acétone.

Le produit attendu recristallise par refroidissement de la solution filtrée, on l'essore sur verre fritté et on le lave deux fois à l'acétone refroidie.

Après séchage, 300 g de poudre blanche (Rendement = 77%) sont obtenus.

b) ANALYSES DU PRODUIT OBTENU.

- Fusion à : 90°C
- RMN $^{13}C$ : conforme à la formule.
- Analyse élémentaire : $C_{20}H_{42}N_2O_7S_2$ PM = 486

| | C | H | O | N | S |
|---|---|---|---|---|---|
| Théorique | 49,38 | 8,64 | 23,05 | 5,76 | 13,17 |
| Trouvé | 49,48 | 8,68 | 22,90 | 5,79 | 13,11 |

EXEMPLES DE FORMULATION

EXEMPLE 1

On prépare un shampooing mousse destiné au traitement de repousse du cheveu, de composition suivante :

| | | |
|---|---|---|
| - Minoxidil | | 4,0 g |
| - Composé B | | 9,7 g |
| - Tensio-actif non ionique poly(hydroxy propyléther) préparé par condensation, en catalyse alcaline, de 3,5 moles de glycidol sur un mélange d'alpha-diols ayant 11 à 14 atomes de carbone, selon le procédé décrit dans le brevet FR-2.091.516 | | 13,0 g |
| - Conservateurs | qs | |
| - Eau | qsp | 100,0 g |

EXEMPLE 2

Pour préparer un conditionnement aérosol de la composition de l'exemple 1, on utilise :

| | |
|---|---|
| - Composition de l'exemple 1 | 95,0 g |
| - Mélange ternaire de N-butane, isobutane > 55%, propane, vendu sous la dénomination "AEROGAZ 3,2N" par la Société ELF AQUITAINE | 5,0 g |

EXEMPLE 3

On prépare une lotion non rincée destinée au traitement de repousse du cheveu, de composition suivante :

| | | |
|---|---|---|
| - Minoxidil | | 2,0 g |
| - Composé B | | 4,85 g |
| - Tensio-actif non ionique poly(hydroxy propyléther) préparé par condensation, en catalyse alcaline, de 3,5 moles de glycidol sur un mélange d'alpha-diols ayant 11 à 14 atomes de carbone, selon le procédé décrit dans le brevet FR-2.091.516 | | 1,0 g |
| - Conservateurs | qs | |
| - Eau | qsp | 100,0 g |

EXEMPLE 4

On prépare un gel non rincé destiné au traitement de repousse du cheveu, de composition suivante :

| | | |
|---|---|---|
| - Minoxidil | | 3,0 g |
| - Composé A | | 9,0 g |
| - Alcool éthylique à 95% vol. | | 20,0 g |
| - Eau | qsp | 100,0 g |

EXEMPLE 5

On prépare la composition de coloration suivante :

| | | | | |
|---|---|---|---|---|
| - Composé A | | | 2,5 | g |
| - N-(ß-hydroxyéthyl)amino-1 nitro-2 N',N'-(bis-ß-hydroxyéthyl)amino-4 benzène | | | 0,8 | g |
| - Triéthanolamine | qs | pH=7 | | |
| - Eau | | qsp | 100,0 | g |

EXEMPLE 6

On prépare la composition de coloration capillaire suivante :

| | | | | |
|---|---|---|---|---|
| - Composé B | | | 2,5 | g |
| - N-(ß-hydroxyéthyl)amino-1 nitro-2 N',N'-(bis-ß-hydroxyéthyl)amino-4 benzène | | | 0,8 | g |
| - Triéthanolamine | qs | pH=7 | | |
| - Eau | | qsp | 100,0 | g |

EXEMPLE 7

On prépare la composition de coloration capillaire suivante :

| | | | | |
|---|---|---|---|---|
| - Composé A | | | 4,0 | g |
| - N-(ß-hydroxyéthyl)amino-1 nitro-2 N'-méthyl N'-(ß-hydroxyéthyl)amino-4 benzène | | | 0,6 | g |
| - Triéthanolamine | qs | pH=7 | | |
| - Eau | | qsp | 100,0 | g |

EXEMPLE 8

On prépare un shampooing antipelliculaire de composition suivante :

| | | | |
|---|---|---|---|
| - Composé A | | 0,6 | g |
| - Alkyl($C_{12}$-$C_{14}$)éthersulfate de sodium oxyéthyléné à 2,2 moles d'oxyde d'éthylène, vendu à 25% de MA | | 7,5 | g |
| - Diéthanolamide d'acide de coprah | | 4,0 | g |
| - Sel d'éthanolamine de l'hydroxy-1 méthyl-4 (triméthyl-2,4,4)-pentyl-6 1H-pyridinone-2, vendu sous la dénomination "OCTOPYROX" par la Société HOECHST | | 0,2 | g |
| - Colorants, parfum, qs | | | |
| - Triéthanolamine qs pH=7 | | | |
| - Eau | qsp | 100,0 | g |

EXEMPLE 9

On prépare un après-shampooing à rincer de composition suivante :

| | | |
|---|---|---|
| - Composé B | 0,5 | g |
| - Hydroxyéthylcellulose | 1,0 | g |
| - Polymère d'hydroxyéthylcellulose et d'épichlorhydrine quaternisé avec la triméthylamine, vendu sous la dénomination "JR 400" par la Société UNION CARBIDE | 0,8 | g |
| - Chlorure de sodium | 4,0 | g |
| - Chlorure de dialkyl($C_{16}$-$C_{18}$/35-65) diméthylammonium | 0,6 | g |

| | | | |
|---|---|---|---|
| - Mélange (80/20) d'alcools cétylstéarylique et cétylstéarylique oxyéthyléné à 33 moles d'oxyde d'éthylène, vendu sous la dénomination "SINNOWAX AO" par la Société HENKEL | | 2,0 | g |
| - Alcool stéarylique | | 1,0 | g |
| - Alcool cétylique | | 1,0 | g |
| - Conservateurs, colorants, qs | | | |
| - Triéthanolamine qs pH=7 | | | |
| - Eau | qsp | 100,0 | g |

EXEMPLE 10

Solubilisation du N-(β-hydroxyéthyl)amino-1 nitro-2 N',N'-(bis-β-hydroxyéthyl)amino-4 benzène, de formule :

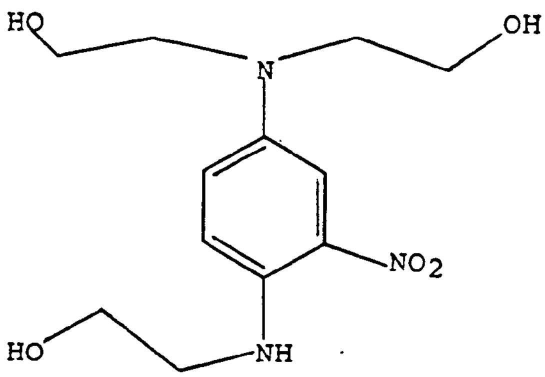

Ce colorant est insoluble dans l'eau à des taux de O,8% en poids à pH7 par la triéthanolamine.
On prépare la composition suivante :

| | | | |
|---|---|---|---|
| - Colorant ci-dessus | | 0,8 | % |
| - Composé A | | 2,5 | % |
| - Triéthanolamine qs pH=7 | | | |
| - Eau | qsp | 100,0 | g |

Le colorant est solubilisé.

Traitement de mèches :

Un couple de mèches de cheveux à 90% blancs et 90% blancs permanentés (2 x 1,5 g) est immergé dans 15 g de solution pendant 30 minutes. Les cheveux sont ensuite rincés et séchés.

| | COULEUR |
|---|---|
| CHEVEUX 90% BLANCS | POURPRE GRISATRE (0,7 RP ; 3,9 ; 1,6) |
| CHEVEUX 90% BLANCS PERMANENTES | POURPRE NOIRATRE (4P ; 2,3 ; 3,3) |

La notation de la couleur a été effectuée selon le système Munsell, les trois données chiffrées indiquant respectivement la teinte, la clarté et la saturation. Les mesures colorimétriques ont été effectuées avec le "Minolta CR 200".

EXEMPLE 11

Dans les mêmes conditions que dans l'exemple 10, on prépare la composition suivante :

- Colorant de l'exemple 10 0,8 %
- Composé B 2,5 %
- Triéthanolamine qs pH=7
- Eau qsp 100,0 g

Dans ces conditions, le colorant 1 est solubilisé.

Traitement de mèches :

Avec le même protocole de traitement que dans l'exemple 10, on obtient les résultats ci-après.

| | COULEUR |
|---|---|
| CHEVEUX 90% BLANCS | POURPRE GRISATRE (1,1 RP ; 3,9 ; 1,8) |
| CHEVEUX 90% BLANCS PERMANENTES | POURPRE NOIRATRE (4P ; 2,2 ; 3,4) |

EXEMPLE 12

Solubilisation de nitro-3 N',β-hydroxyéthylamino 4-N-méthyl N-β-hydroxyéthylaniline, de formule :

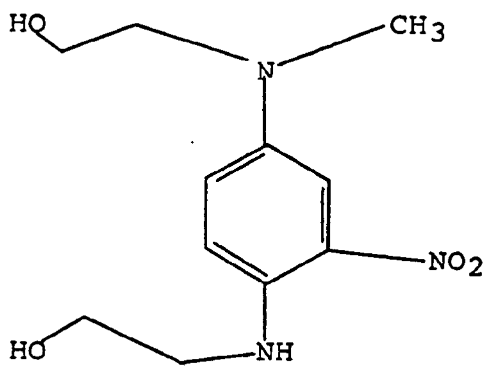

Ce colorant est insoluble dans l'eau à 0,6% en poids à pH7 par la triéthanolamine.
On réalise sa solubilisation en préparant la composition suivante :

| | | |
|---|---|---|
| - Colorant ci-dessus | | 0,6 % |
| - Composé A | | 4,0 % |
| - Triéthanolamine qs pH=7 | | |
| - Eau | qsp | 100,0 g |

Traitement de mèches :

Avec le même protocole de traitement que dans l'exemple 10, on obtient les résultats suivants :

| | COULEUR |
|---|---|
| CHEVEUX 90% BLANCS | POURPRE GRISATRE FONCE (0,1 RP ; 3,3 ; 2,6) |
| CHEVEUX 90% BLANCS PERMANENTES | POURPRE NOIRATRE (5,3 P ; 2,1 ; 3,4) |

**Revendications**

1. Composés de formule générale (I) :

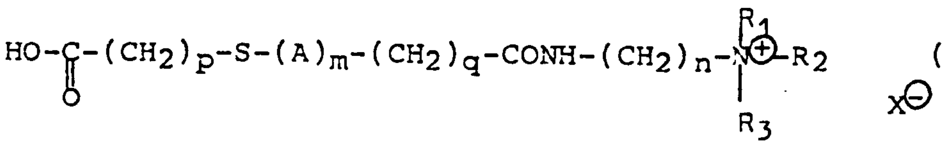 (I)

dans laquelle :

$R_1$ et $R_2$, identiques ou différents, désignent un radical méthyle ou éthyle;

$R_3$ désigne un radical méthyle, éthyle ou hydroxyéthyle;

A désigne un radical

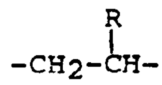 ou 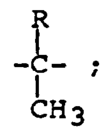 ;

R désigne un atome d'hydrogène ou un radical méthyle;

$X^-$ désigne $Cl^-$,$Br^-$,$I^-$,$CH_3OSO_3^-$,$CH_3SO_3^-$,

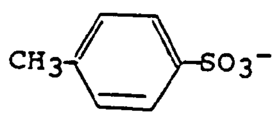

ou $C_2H_5OSO_3^-$;

p et q, identiques ou différents, désignent des nombres entiers tels que $1 \leqq p \leqq 15$, $O \leqq q \leqq 13$ et $2 \leqq p+q \leqq 20$; et

m désigne 0 ou 1 et $m+q \neq 0$;

n désigne un nombre entier égal à 2 ou 3,

ainsi que leurs sels de neutralisation par des bases.

**2.** Composés selon la revendication 1, caractérisés en ce que dans la formule (I), $R_1$ et/ou $R_2$ désignent un radical méthyle.

**3.** Composés selon la revendication 1 ou 2, caractérisés en ce que p = 1 ou 10, q = 10, m = 0, n = 3, $R_1$, $R_2$ et $R_3$ désignent un radical méthyle et $X^-$ désigne $CH_3OSO_3^-$.

**4.** Composés selon l'une des revendications 1 à 3, caractérisés en ce que les bases de neutralisation sont choisies parmi la soude, la potasse, l'ammoniaque, la magnésie, la diméthyléthanolamine, les aminométhyl propanols, les aminométhylpropanediols, la triéthanolamine et la N-méthylglucamine.

**5.** Procédé de préparation des composés de formule (I) tels que définis à la revendication 1, caractérisé en ce qu'il comporte au moins la réaction d'addition sur le composé correspondant de formule (III) :

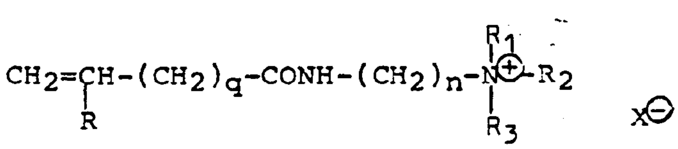 (III)

du mercaptoacide correspondant de formule (II) :

$$HOOC\text{-}(CH_2)_p\text{-}SH \qquad (II)$$

dans lesquelles $R_1$, $R_2$, $R_3$, R, $X^-$, n, p et q sont définis comme dans la revendication 1,

éventuellement en présence de catalyseur, dans un solvant approprié,

et éventuellement la neutralisation du produit obtenu.

**6.** Procédé selon la revendication 5, caractérisé en ce que l'addition est radicalaire et réalisée en présence

d'initiateur de radicaux libres.

7. Procédé selon la revendication 5 ou 6, caractérisé en ce que l'initiateur de radicaux libres est l'azobis isobutyronitrile.

8. Utilisation des composés de formule (I) tels que définis à la revendication 1 comme additifs dans des compositions de traitement des matières kératiniques.

9. Utilisation des composés de formule (I) tels que définis à la revendication 1, comme solubilisants et/ou dispersants dans des solutions aqueuses ou hydroalcooliques.

10. Composition aqueuse ou hydroalcoolique pour l'application topique, caractérisée en ce qu'elle comporte au moins un composé de formule (I) telle que définie à la revendication 1.

11. Composition pour le traitement des matières kératiniques, caractérisée en ce qu'elle comporte au moins un composé de formule (I) telle que définie à la revendication 1.

12. Composition cosmétique, caractérisée en ce qu'elle comporte au moins un composé de formule (I) telle que définie à la revendication 1.

13. Composition dermopharmaceutique, caractérisée en ce qu'elle comporte au moins un composé de formule (I) telle que définie à la revendication 1.

14. Composition selon l'une quelconque des revendications 10 à 13, caractérisée en ce qu'elle comporte de 0,1 à 10% en poids desdits composés, de préférence 0,3 à 5% en poids.

15. Composition selon l'une quelconque des revendications 10 à 14, caractérisée en ce qu'elle comporte en outre un ou plusieurs colorants des matières kératiniques, une ou des cires, une ou plusieurs huiles minérales, animales, végétales ou synthétiques telles que les huiles de silicone un ou plusieurs polymères d'origine naturelle, cellulosique ou dérivés de chitine ou de chitosane, un ou plusieurs tensio-actifs ioniques ou non-ioniques, un ou plusieurs filtres solaires, un ou plusieurs conservateurs, un ou plusieurs agents actifs pour la repousse des cheveux, un ou plusieurs agents actifs pour le traitement des pellicules, un ou plusieurs agents actifs pour le traitement de l'acné, un ou plusieurs agents épaississants ou gélifiants, un ou plusieurs agents nacrants, un ou plusieurs agents hydratants, un ou plusieurs solvants tels que les alcools en $C_2$ à $C_5$, les éthers de glycol, un ou plusieurs propulseurs tels que les fréons, les alcanes en $C_3$ à $C_5$, le chlorure de méthylène et le diméthyl éther, et un ou plusieurs stérols, ou leurs mélanges.

16. Composition selon l'une quelconque des revendications 10 à 15, caractérisée en ce qu'elle comporte du minoxidil.

17. Utilisation de la composition selon l'une quelconque des revendications 10 à 12 ou 14 à 16, pour le traitement cosmétique des cheveux, poils, ongles ou de la peau.

## Patentansprüche

1. Verbindungen der allgemeinen Formel (I) :

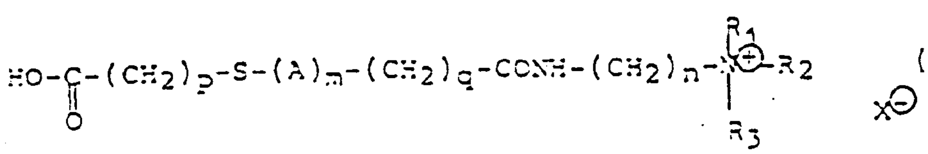

(I)

worin:
$R_1$ und $R_2$, die gleich oder verschieden sind, ein Methyl- oder Ethylradikal bedeuten;
$R_3$ ein Methyl-, Ethyl- oder,Hydroxyethylradikal bedeutet;
A ein Radikal

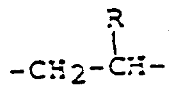

oder

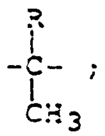

R ein Wasserstoffatom oder ein Methylradikal bedeutet;
$X^-$ $Cl^-$, $Br^-$, $I^-$, $CH_3OSO_3^-$, $CH_3SO_3^-$,

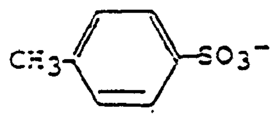

oder
$C_2H_5OSO_3^-$;
darstellt;
p und q gleich oder verschieden sind und eine ganze Zahl bedeuten, wobei $1 \leqq p \leqq 15$, $0 \leqq q \leqq 13$ und $2 \leqq p+q \leqq 20$ ist;
m 0 oder 1 bedeutet, und $m + q \neq 0$;
n eine ganze Zahl bedeutet, die 2 oder 3 ist;
sowie ihre Neutralisationssalze mit Basen.

2. Verbindungen gemäß Anspruch 1, dadurch gekennzeichnet, daß in Formel (I) $R_1$ und/oder $R_2$ ein Methylradikal bedeuten.

3. Verbindungen gemäß Anspruch 1 oder 2, dadurch gekennzeichnet, daß p = 1 oder 10, Q = 10, m = 0, n = 3, $R_1$, $R_2$ und $R_3$ ein Methylradikal bedeuten, und $X^-$ $CH_3OSO_3^-$ bedeutet.

4. Verbindungen gemäß einem der Ansprüche 1 bis 3, dadurch gekennzeichnet, daß die Neutralisationsbasen ausgewählt sind unter Natriumhydroxid, Kaliumhydroxid, Ammoniak, Magnesiumoxid, Dimethylethylamin, den Aminomethylpropanolen, den Aminomethylpropandiolen, Triethanolamin und N-Methylglucamin.

5. Verfahren zur Herstellung von Verbindungen der Formel (I) gemäß Anspruch 1, dadurch gekennzeichnet, daß es mindestens umfaßt die Additionsreaktion einer entsprechenden Mercaptosäure der Formel (III) :

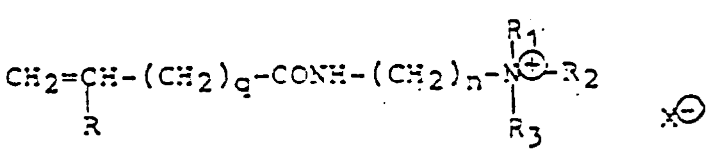
(III)

an der entsprechenden Verbindung der Formel (II) :

$$HOOC\text{-}(CH_2)_p\text{-}SH \qquad (II)$$

worin $R_1$, $R_2$, $R_3$, R, $X^-$, n, p und q die in Anspruch 1 angegebene Bedeutung besitzen, gegebenenfalls in Gegenwart eines Katalysators, in einem geeigneten Lösungsmittel, und gegebenenfalls die Neutralisation des erhaltenen Produktes.

6. Verfahren gemäß Anspruch 5, dadurch gekennzeichnet, daß die Addition vom Radikaltyp ist und in Gegenwart eines Initiators für freie Radikale durchgeführt wird.

7. Verfahren gemäß Anspruch 5 oder 6, dadurch gekennzeichnet, daß der Initiator für freie Radikale Azobisisobutyronitril ist.

8. Verwendung der Verbindungen der Formel (I) gemäß Anspruch 1 als Additive in Behandlungszusammensetzungen für keratinische Materialien.

9. Verwendung der Verbindungen der Formel (I) gemäß Anspruch 1 als löslich machende und/oder dispergierende Verbindungen in wässerigen oder wässerig-alkoholischen Lösungen.

10. Wässerige oder wässerig-alkoholische Zusammensetzung zur topischen Applikation, dadurch gekennzeichnet, daß sie mindestens eine Verbindung der Formel (I) gemäß Anspruch 1 enthält.

11. Zusammensetzung zur Behandlung von Keratinmaterialien, dadurch gekennzeichnet, daß sie mindestens eine Verbindung der Formel (I) gemäß Anspruch 1 enthält.

12. Kosmetische Zusammensetzung, dadurch gekennzeichnet, daß sie mindestens eine Verbindung der Formel (I) gemäß Anspruch 1 enthält.

13. Dermopharmazeutische Zusammensetzung, dadurch gekennzeichnet, daß sie mindestens eine Verbindung der Formel (I) gemäß Anspruch 1 enthält.

14. Zusammensetzung gemäß einem der Ansprüche 10 bis 13, dadurch gekennzeichnet, daß sie 0.1 bis 10 Gew.-% der Verbindungen, vorzugsweise 0.3 bis 5 Gew.-%, enthält.

15. Zusammensetzung nach einem der Ansprüche 10 bis 14, dadurch gekennzeichnet, daß sie außerdem ein oder mehrere Farbstoffe für Keratinmaterialien, ein oder mehrere Wachse, ein oder mehrere Mineralöle, tierische Öle, pflanzliche Öle oder synthetische Öle, wie z.B. Siliconöle, enthält, und ein oder mehrere natürliche Cellulose-Polymere oder Derivate von Chitin oder Chitosan, ein oder mehrere oberflächenaktive ionische oder nicht-ionische Stoffe, ein oder mehrere Sonnenfilter, ein oder mehrere Konservierungsmittel, ein oder mehrere Haarwuchsmittel, ein oder mehrere Schuppenbehandlungsmittel, ein oder mehrere Acnebehandlungsmittel, ein oder mehrere Verdickungsmittel oder Gelierungsmittel, ein oder mehrere Glanzmittel, ein oder mehrere Hydratisierungsmittel, ein oder mehrere Lösungsmittel, wie z.B. $C_2$-$C_5$ Alkohole, Glycolether, ein oder mehrere Treibmittel, wie z.B. die Freone, die $C_2$-$C_5$-Alkane, Methylenchlorid und Dimethylether, ein oder mehrere Sterole, oder ihre Mischungen enthält.

16. Zusammensetzung nach einem der Ansprüche 10 bis 15, dadurch gekennzeichnet, daß sie Minoxidil enthält.

17. Verwendung einer Zusammensetzung gemäß einem der Ansprüche 10 bis 12 oder 14 bis 16 zur kosmetischen Behandlung von Haaren, Fellen, Nägeln oder der Haut.

## Claims

1. Compounds of general formula (I) :

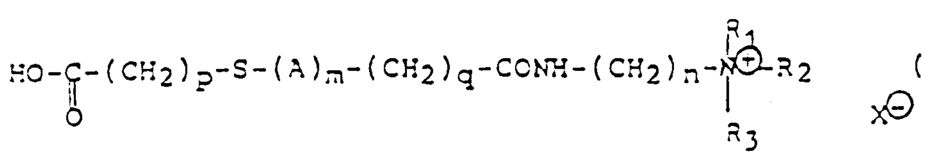

(I)

in which:

$R_1$ and $R_2$, which are identical or different, denote a methyl or ethyl radical;
$R_3$ denotes a methyl, ethyl or hydroxyethyl radical;
A denotes a

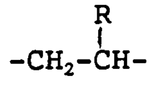

radical or

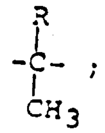

R denotes a hydrogen atom or a methyl radical;

$X^-$ denotes $Cl^-$, $Br^-$, $I^-$, $CH_3OSO_3^-$, $CH_3SO_3^-$,

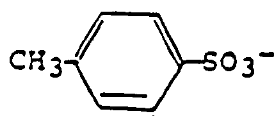

or $C_2H_5OSO_3^-$;

p and q, which are identical or different, denote integers such that $1 \leqq p \leqq 15$, $0 \leqq q \leqq 13$ and $2 \leqq p+q \leqq 20$;

n denotes an integer equal to 2 or 3;

m denotes 0 or 1, and $m + q \neq 0$;

as well as their salts from neutralisation by bases.

2. Compounds according to Claim 1, characterised in that, in formula (I), $R_1$ and/or $R_2$ denote a methyl radical.

3. Compounds according to Claim 1 or 2, characterised in that p = 1 or 10, q = 10, m = 0, n = 3, $R_1$, $R_2$ and $R_3$ denote a methyl radical and $X^-$ denotes $CH_3OSO_3^-$.

4. Compounds according to one of Claims 1 to 3, characterised in that the bases for neutralisation are chosen from amongst sodium hydroxide, potassium hydroxide, ammonia, magnesium hydroxide, dimethylethanolamine, aminomethylpropanols, aminomethylpropanediols, triethanolamine and N-methylglucamine.

5. Process for preparation of the compounds of formula (I) such as defined in Claim 1, characterised in that it comprises at least the addition reaction with the corresponding compound of formula (III) :

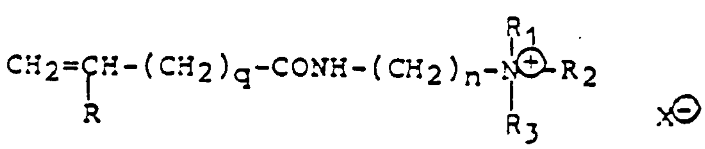
(III)

of the corresponding mercapto acid of formula (II) :

$$HOOC\text{-}(CH_2)_p\text{-}SH \qquad (II)$$

in which $R_1$, $R_2$, $R_3$, R, $X^-$, n, p and q are defined as in Claim 1,

if appropriate in the presence of catalyst, in an appropriate solvent,

and if appropriate neutralising the product obtained.

6. Process according to Claim 5, characterised in that the addition is a radical addition and is carried out in the presence of a free radical initiator.

7. Process according to Claim 5 or 6, characterised in that the free radical initiator is azobisisobutyronitrile.

8. Use of the compounds of formula (I) such as defined in Claim 1 as additives in compositions for treatment of keratinous materials.

9. Use of the compounds of formula (I) such as defined in Claim 1 as solubilisers and/or dispersants in aqueous or aqueous-alcoholic solutions.

10. Aqueous or aqueous-alcoholic composition for topical application, characterised in that it comprises at least one compound of formula (I) such as defined in Claim 1.

**11.** Composition for the treatment of keratinous materials, characterised in that it comprises at least one compound of formula (I) such as defined in Claim 1.

**12.** Cosmetic composition, characterised in that it comprises at least one compound of formula (I) such as defined in Claim 1.

**13.** Dermopharmaceutical composition, characterised in that it comprises at least one compound of formula (I) such as defined in Claim 1.

**14.** Composition according to any one of Claims 10 to 13, characterised in that it comprises from 0.1 to 10% by weight of the said compounds, preferably 0.3 to 5% by weight.

**15.** Composition according to any one of Claims 10 to 14, characterised in that it additionally comprises one or more colorants of keratinous materials, a wax or waxes, one or more mineral, animal, vegetable or synthetic oils such as silicone oils, one or more polymers of natural origin, cellulosic polymers or derivatives of chitin or chitosan, one or more ionic or non-ionic surfactants, one or more sunscreens, one or more preservatives, one or more active agents for the regrowth of hair, one or more active agents for the treatment of dandruff, one or more active agents for the treatment of acne, one or more thickening or gelling agents, one or more pearlising agents, one or more hydrating agents, one or more solvents such as $C_2$ to $C_5$ alcohols or glycol ethers, one or more propellants such as Freons, $C_3$ to $C_5$ alkanes, methylene chloride and dimethyl ether, and one or more sterols, or their mixtures.

**16.** Composition according to any one of Claims 10 to 15, characterised in that it comprises minoxidil.

**17.** Use of the composition according to any one of Claims 10 to 12 or 14 to 16 for the cosmetic treatment of the hair, nails or skin.